# EUROPÄISCHE PATENTSCHRIFT

(11) **EP 2 520 236 B1**
(45) Veröffentlichungstag und Bekanntmachung des Hinweises auf die Patenterteilung: **15.07.2015**
(21) Anmeldenummer: 11003618.3
(22) Anmeldetag: 03.05.2011
(51) Int. Cl.: A61B 17/32

(54) **Rotierendes chirugisches Instrument**
Rotating surgical instrument
Instrument chirurgical rotatif

(43) Veröffentlichungstag der Anmeldung: 07.11.2012
(73) Patentinhaber: Eberle GmbH & Co. KG, 75449 Wurmberg (DE)
(72) Erfinder: Amann, Bernd, 75449 Wurmberg (DE)
(74) Vertreter: Hoefer & Partner Patentanwälte mbB

(56) Entgegenhaltungen:
- US-A1- 2005 054 972

## Beschreibung

Die vorliegende Erfindung betrifft ein rotierendes chirurgisches Instrument.

Chirurgische Instrumente werden in der Medizin beispielsweise zum Entfernen von Körpermaterial, z.B. Gewebe oder Knochen oder dgl., verwendet.

Aus der WO 2010/118831 A1 ist beispielsweise ein chirurgisches Instrument bekannt, welches ein starres Außenrohr und ein starres Innenrohr aufweist, welche demontierbar sind. Dieses Instrument hat sich grundsätzlich bewährt, allerdings gibt es Anwendungsfälle, bei denen es notwendig ist, dass das chirurgische Instrument teilweise flexibel ist.

Aus der DE 28 13 750 ist ein medizinisches Instrument aus Kunststoff bekannt, welches mehrere poröse Bereiche aufweist, um eine Flexibilität des Instruments bereitzustellen. Dieses Instrument ist jedoch insbesondere zum Einbringen oder Entnehmen von Flüssigkeiten in einen Körper vorgesehen. Ferner zeigt die US 2005/0054972A1 ein chirurgisches Instrument gemäß dem Oberbegriff des Anspruchs 1.

Es ist daher Aufgabe der vorliegenden Erfindung, ein chirurgisches Instrument bereitzustellen, welches bei einfachem und kostengünstigen Aufbau eine gewisse Flexibilität aufweist, um Körpermaterial auch an schwierig zu erreichenden Stellen in einem Körper entfernen zu können.

Diese Aufgabe wird durch ein chirurgisches Instrument mit den Merkmalen des Anspruches 1 gelöst. Die Unteransprüche zeigen bevorzugte Weiterbildungen der Erfindung.

Das erfindungsgemäße chirurgische Instrument weist ein rotierbares, rohrförmiges Schneidrohr mit einem Schneidelement an einem freien Ende und ein das Schneidrohr umgebendes Zwischenrohr auf. Ferner ist ein Führungsrohr vorgesehen, welches das Zwischenrohr umgibt. Das Führungsrohr ist an einem Endbereich mit einem Rohrhalter verbunden und das Zwischenrohr ist mit einem Endbereich an einem Schneidenhalter fixiert. Ferner weist das Schneidrohr einen ersten flexiblen Teilbereich auf, welcher sich vorzugsweise direkt an das Schneidelement anschließt. Das Zwischenrohr weist einen zweiten flexiblen Teilbereich auf, welcher in Längsrichtung des chirurgischen Instruments im gleichen Bereich wie der erste flexible Teilbereich am Schneidrohr gebildet ist. Das Schneidrohr und das Zwischenrohr sind ferner relativ zum Führungsrohr in Längsrichtung des chirurgischen Instruments verschiebbar. Ferner weist das Führungsrohr einen zungenförmigen, gebogenen freien Endbereich auf, welcher beim Herausschieben den ersten und zweiten flexiblen Teilbereich des Schneidrohrs und des Zwischenrohrs führt. Um das Schneidrohr anzutreiben, ist das Schneidrohr derart eingerichtet, dass es mit einem Antrieb verbindbar ist. Durch die erfindungsgemäße Ausgestaltung ist es somit möglich, dass das Schneidrohr gemeinsam mit dem Zwischenrohr aus dem Führungsrohr in Längsrichtung des chirurgischen Instruments herausgeschoben bzw. zurückgezogen werden kann. Durch die flexiblen Teilbereiche können dabei das Schneidrohr und das Zwischenrohr auch an schwierig zu erreichende Körperstellen gebracht werden. Trotzdem kann das erfindungsgemäße chirurgische Instrument einfach in einen Körper eingebracht werden, da während des Einbringvorgangs das Schneidrohr und das Zwischenrohr vollständig im Inneren des steifen Führungsrohrs angeordnet sein können und dann an der Operationsstelle aus dem Führungsrohr herausgeschoben werden können und durch die Führungszunge geführt werden.

Vorzugsweise weist das Schneidrohr eine Absaugöffnung auf. Die Absaugöffnung ist vorzugsweise an oder nahe dem Schneidelement des Schneidrohrs angeordnet. Hierdurch ist es möglich, dass abgetrenntes Gewebe oder dgl. durch die Absaugöffnung in das Innere des hohlen Schneidrohrs abgesaugt werden kann und durch das hohle Schneidrohr aus dem Körper gebracht werden kann.

Weiter bevorzugt ist die Führungszunge vollständig in einem durch einen Außenumfang des Führungsrohrs definierten zylindrischen Umrisskörper angeordnet. Hierdurch wird vermieden, dass die Führungszunge zu weit vom chirurgischen Instrument vorsteht, was ein Einführen des chirurgischen Instruments zu einer Operationsstelle erschweren könnte.

Gemäß einer weiteren bevorzugten Ausgestaltung der vorliegenden Erfindung ist der Schneidehalter mit dem Rohrhalter mittels wenigstens eines Stiftes, vorzugsweise mittels zwei Stiften, verbunden. Hierbei ist der Rohrhalter relativ zum Schneidenhalter beweglich vorgesehen, wodurch das Schneidrohr und das Zwischenrohr in und aus dem Führungsrohr herausgeschoben bzw. zurückgezogen werden kann. Die Stifte übernehmen dabei insbesondere auch eine Führungsfunktion für eine sichere und robuste Verstellung der Position von Schneidrohr und Zwischenrohr.

Vorzugsweise ist der Schneidenhalter mit dem Rohrhalter verdrehsicher verbunden. Weiter bevorzugt ist zwischen dem Schneidenhalter und dem Rohrhalter eine lösbare Verriegelung vorgesehen. Hierdurch kann eine jeweils gewählte Relativposition zwischen dem Schneidenhalter und dem Rohrhalter fixiert werden, so dass es während einer Operation nicht zu Relativbewegungen zwischen diesen beiden Bauteilen kommt. Hierdurch kann insbesondere eine unbeabsichtigte Lageänderung des Schneidelements vermieden werden. Die Verriegelungseinrichtung fixiert somit eine herausgeschobene Arbeitsposition des Schneidrohrs. Die Verriegelung ist vorzugsweise ein manuell betätigbares Klemmelement.

Vorzugsweise weist der erste und/oder zweite flexible Teilbereich des Schneidrohrs bzw. des Zwischenrohrs eine Vielzahl von Schlitzen auf. Hierdurch kann auf einfache und kostengünstige Weise ein flexibler Teilbereich bereitgestellt werden. Am Zwischenrohr sind die Schlitze vorzugsweise nur an einer Umfangsseitenhälfte vorgesehen. Am Schneidrohr sind die Schlitze abwechselnd an jeweils einander gegenüberliegenden Seitenumfangsflächen vorgesehen, da das Schneidrohr rotiert und somit in alle Richtungen flexibel sein muss. Erfindungsgemäß wird unter dem Begriff "Schlitz" auch ein einziger, mäandrierender Schlitz verstanden.

Um ein möglichst einfaches Einführen des chirurgischen Instruments zu ermöglichen, ist der zungenförmige, gebogene erste Endbereich des Führungsrohrs mit einer abgerundeten Spitze gebildet. Der zungenförmige Endbereich weist vorzugsweise einen Radius zwischen 20 bis 30 mm, vorzugsweise ca. 25 mm, auf.

Besonders bevorzugt ist der erste flexible Teilbereich des Schneidrohrs derart angeordnet, dass sich der erste flexible Teilbereich direkt an das Schneidelement anschließt. Somit wird sichergestellt, dass lediglich die Spitze des chirurgischen Instruments flexibel ist, was ein besonders exaktes Operieren ermöglicht.

Weiter bevorzugt umfasst das Instrument eine Lagerhülse, welche an einem freien Endbereich des Außenrohrs angeordnet ist, um das Schneidrohr zu lagern. Hierdurch kann das Schneidrohr nahe seinem Schneidelement rotierbar gelagert werden, so dass ein besonders ruhiger Lauf des Schneidrohrs erhalten wird.

Um eine möglichst schnelle Verbindung zwischen dem chirurgischen Instrument und einem Antriebsteil, beispielsweise einem mit Motor versehenen Handstück, zu gewährleisten, weist ein zweiter Endbereich des Schneidrohrs eine Kupplung auf. Über diese Kupplung kann das chirurgische Instrument an den Antrieb angeschlossen werden und die vom Antrieb erzeugten Rotationskräfte auf das Schneidrohr übertragen werden.

Um eine möglichst gute Reinigung und ein mehrfaches Verwenden der Teile des chirurgischen Instruments zu ermöglichen, ist das chirurgische Instrument vorzugsweise zerlegbar.

Das Schneidelement des Schneidrohrs ist vorzugsweise ein Fräser oder ein Schneidkopf für Gewebematerial. Mit dem Fräser können beispielsweise härtere Materialien wie Knochen oder dgl. entfernt werden. Mit dem Schneidkopf, welcher vorzugsweise bei Draufsicht eine U-förmige Gestalt mit einer Vielzahl von Zähnen aufweist, kann Gewebematerial, z.B. vom Meniskus, entfernt werden.

Das Schneidrohr, das Zwischenrohr und das Führungsrohr, sind vorzugsweise aus Edelstahl hergestellt. Weiter bevorzugt ist zwischen dem Zwischenrohr und dem Schneidenhalter und/oder zwischen dem Führungsrohr und dem Rohrhalter, jeweils eine Schweißverbindung vorgesehen.

Bevorzugt umfasst das erfindungsgemäße chirurgische Instrument ferner ein Zugangsrohr, welches das Führungsrohr umgibt. Hierdurch können das Schneidrohr, das Zwischenrohr und das Führungsrohr im Zugangsrohr, welches einen Zugang zum Körper eines Patienten ermöglicht, verfahren werden und gegebenenfalls auch vollständig aus dem Zugangsrohr entfernt werden und ein anderes chirurgisches Instrument über das Zugangsrohr eingeführt werden.

Besonders bevorzugt ist insbesondere aus hygienischen Gründen die Führungszunge und das Führungsrohr als einstückiges Bauteil ohne Schweißverbindung oder dgl. hergestellt. Dies kann beispielsweise durch Bearbeiten eines freien Endes des Führungsrohres erhalten werden, aus welchem die Führungszunge herausgearbeitet wird.

Nachfolgend werden unter Bezugnahme auf die begleitende Zeichnung bevorzugte Ausführungsbeispiele der Erfindung im Detail beschrieben. In der Zeichnung ist:
- Fig. 1: eine schematische Seitenansicht eines erfindungsgemäßen chirurgischen Instruments im eingezogenen Zustand,
- Fig. 2: eine schematische Seitenansicht des Instruments von Fig. 1 im ausgerückten Zustand,
- Fig. 3: eine schematische Schnittansicht des Instruments von Fig. 1 im eingezogenen Zustand,
- Fig. 4: eine schematische Schnittansicht des Instruments im ausgerückten Zustand,
- Fig. 5: eine vergrößerte Schnittansicht des distalen Endes des chirurgischen Instruments im eingezogenen Zustand,
- Fig. 6: eine schematische Schnittansicht des distalen Endes des chirurgischen Instruments im ausgerückten Zustand,
- Fig. 7: eine schematische Draufsicht auf das chirurgische Instrument im ausgerückten Zustand,
- Fig. 8: eine schematische Unteransicht von unten von Fig. 7,
- Fig. 9: eine schematische Ansicht des chirurgischen Instruments von Fig. 7 von vorne,
- Fig. 10: eine schematische Schnittansicht eines distalen Endes eines chirurgischen Instruments gemäß einem zweiten Ausführungsbeispiel der Erfindung, und
- Fig. 11: eine schematische Draufsicht auf das chirurgische Instrument von Fig. 10.

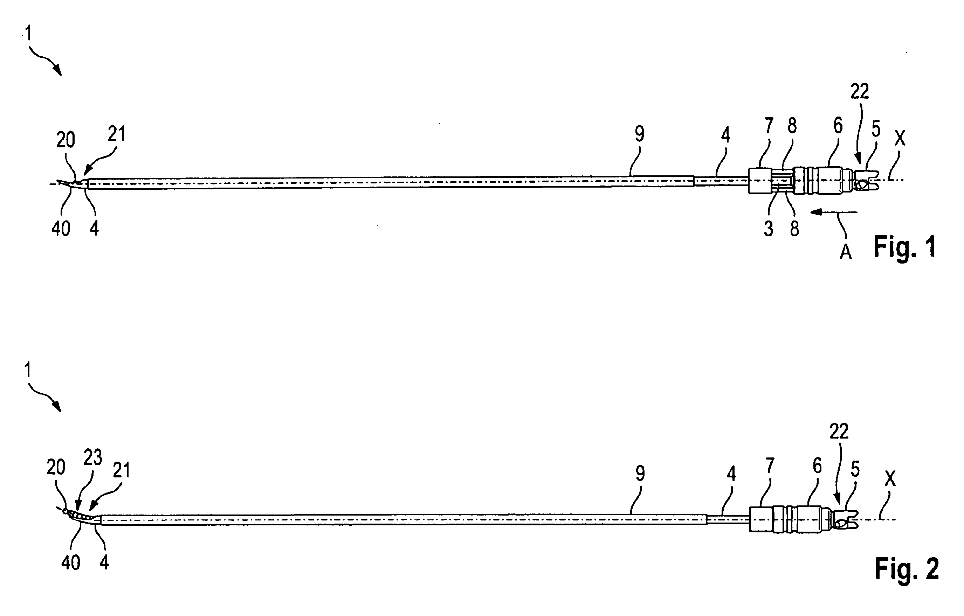

Nachfolgend wird unter Bezugnahme auf die Fig. 1 bis 9 ein chirurgisches Instrument 1 gemäß einem ersten bevorzugten Ausführungsbeispiel der Erfindung im Detail beschrieben.

Wie aus den Gesamtansichten der Fig. 1 und 2 ersichtlich ist, umfasst das chirurgische Instrument 1 mehrere ineinander gesteckte rohrförmige Elemente, einen Schneidenhalter 6, einen Rohrhalter 7 und eine Kupplung 5. Fig. 1 zeigt den zurückgezogenen Zustand des chirurgischen Instruments und Fig. 2 zeigt den ausgerückten bzw. vorgeschobenen Zustand. Wie insbesondere aus Fig. 1 ersichtlich ist, ist der Rohrhalter 7 über zwei Stifte 8 mit dem Schneidenhalter 6 verbunden. Dabei kann der Rohrhalter 7 festgehalten werden und der Schneidenhalter mitsamt der daran angeordneten Kupplung 5 in Richtung des Pfeils A bewegt werden, um das chirurgische Instrument in den vorgeschobenen Zustand zu bringen, in welchem eine Operation erfolgen kann. Die Stifte 8 sind fest im Rohrhalter 7 angeordnet und können in entsprechend gebildete Öffnungen im Schneidenhalter 6 eingeschoben werden.

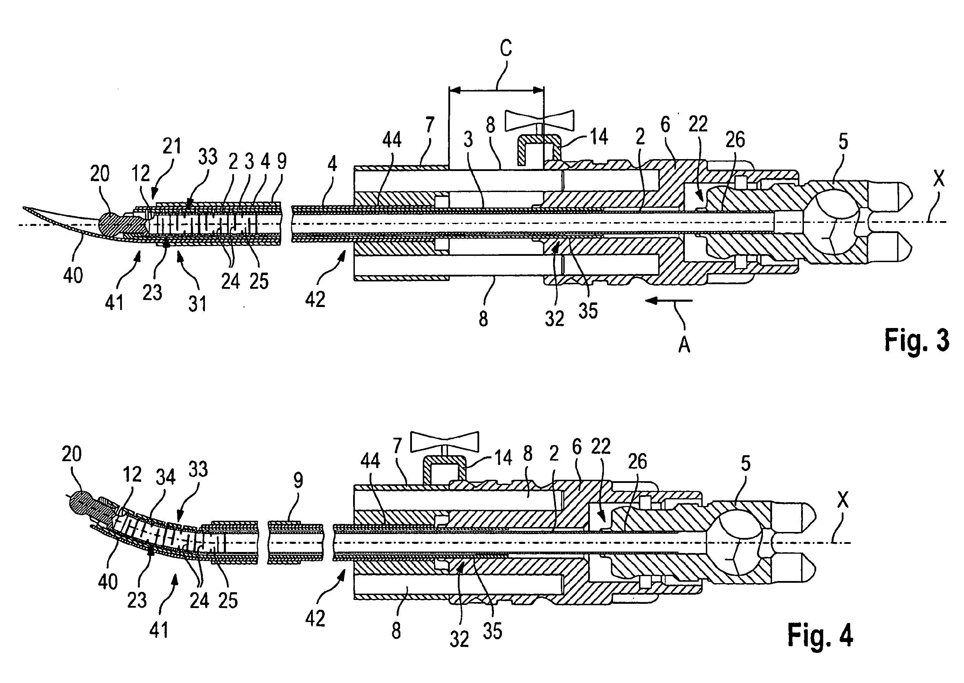

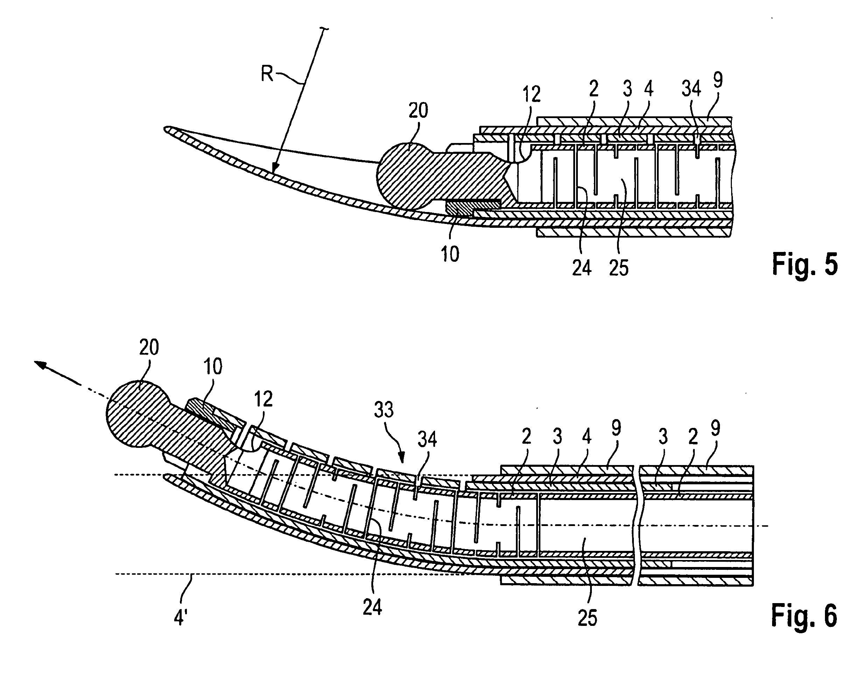

Der rohrförmige Teil des chirurgischen Instruments 1 wird insbesondere aus den Fig. 3 bis 6 ersichtlich. Das chirurgische Instrument 1 umfasst dabei ein Schneidrohr 2, welches als innerstes Rohr angeordnet ist. Das Schneidrohr 2 weist an einem ersten Endbereich 21 ein Schneidelement 20 in Form eines Fräsers auf und an einem zweiten Endbereich 22 ist das Schneidrohr 2 mittels einer Schweißverbindung 26 an der Kupplung 5 angeschweißt. Das Schneidrohr 2 ist hohl und bildet einen Hohlraum 25. Ferner weist das Schneidrohr 2 einen ersten flexiblen Teilbereich 23 auf, welcher benachbart zum Schneidelement 20 vorgesehen ist. Dies ist im Detail aus Fig. 6 ersichtlich. Der flexible Teilbereich 23 wird dabei durch eine Vielzahl von Schlitzen 24 bereitgestellt. Die Schlitze sind abwechselnd an einander gegenüberliegenden Umfangsflächen des Schneidrohrs über einen Winkelbereich von ca. 180° gebildet, um eine Rotationsfähigkeit des flexiblen Teilbereichs 23 sicherzustellen. Das Schneidrohr 2 kann ferner über die Kupplung 5 mit einem Antrieb verbunden werden, so dass es in eine beliebige Drehrichtung rotieren kann.

Ferner umfasst das chirurgische Instrument 1 ein Zwischenrohr 3, welches das Schneidrohr 2 umgibt. Das Zwischenrohr 3 dreht sich nicht und weist an einem ersten Endbereich 31 einen zweiten flexiblen Teilbereich 33 auf. Der flexible Teilbereich 33 wird durch eine Vielzahl von Schlitzen 34 bereitgestellt, wobei die Schlitze 34 nur an einer Umfangshälfte des Zwischenrohrs 3 vorgesehen sind. Wie insbesondere aus Fig. 6 ersichtlich ist, umgibt der zweite flexible Teilbereich 33 dabei den ersten flexiblen Teilbereich 23 des Schneidrohrs 2. Dadurch sind die beiden Rohre in gleicher Weise flexibel ausgebildet. An einem zweiten Endbereich 32 ist das Zwischenrohr 3 mittels einer Schweißverbindung 35 am Schneidenhalter 6 fixiert.

Weiterhin umfasst das chirurgische Instrument 1 ein Führungsrohr 4, welches das Zwischenrohr 3 umgibt. Dadurch liegt in radialer Richtung des chirurgischen Instruments das Zwischenrohr 3 zwischen dem Schneidrohr 2 und dem Führungsrohr 4. Das Führungsrohr 4 umfasst an einem ersten Endbereich 41 eine Führungszunge 40. Die Führungszunge 40 ist insbesondere aus den Fig. 6 bis 8 ersichtlich. Die Führungszunge 40 ist in einem Bogen mit einem Radius R von ca. 25 mm gebildet. Das Führungsrohr 4 ist ferner an einem zweiten Endbereich 42 mittels einer Schweißverbindung 44 am Rohrhalter 7 fixiert.

Weiterhin umfasst das chirurgische Instrument 1 des ersten Ausführungsbeispiels ein Zugangsrohr 9, welches das Führungsrohr 4 teilweise umgibt. Das Zugangsrohr 9 ist nicht zwingend Teil des erfindungsgemäßen chirurgischen Instruments, sondern kann auch einem ersten Schritt in einen Körper eingebracht werden. Das Zugangsrohr 9 dient dabei dazu, dass beispielsweise verschiedene chirurgische Instrumente in einen Körper eingeführt und entnommen werden können, ohne dass jeweils neue Öffnungen im Körper vorgesehen werden müssen. Das Zugangsrohr 9 ist dabei im Wesentlichen ein gerades, zylindrisches Rohr.

Ferner umfasst das chirurgische Instrument eine Verriegelungseinrichtung 14, welche eine Relativposition zwischen dem Schienenhalter 6 und dem Rohrhalter 7 fixieren kann. Hierbei kann die Verriegelungseinrichtung 14 z.B. die zwischen dem Schienenhalter 6 und Rohrhalter 7 vorgesehen Stifte klemmen, so dass jeweils eine vorbestimmte, gewünschte Position erreicht werden kann. Dies ist deshalb wichtig, da dadurch eine ausgefahrene Position des chirurgischen Instruments in exakt dieser Stellung beibehalten werden kann und durch die Bearbeitung von Gewebe oder dgl. nicht ungewünscht verändert werden kann. Es sei angemerkt, dass hierbei jede Position zwischen dem Schneidenhalter 6 und dem Rohrhalter 7 fixiert werden kann. In diesem Ausführungsbeispiel ist die Verriegelungseinrichtung 14 ein manuell betätigbares Klemmelement.

Wie vorher schon erläutert, ist das im Betrieb rotierende Schneidrohr 2 hohl, so dass sich ein Hohlraum 25 ergibt, welcher sich in Längsrichtung X des chirurgischen Instruments bis zur Kupplung 5 erstreckt. Am ersten Endbereich 21 unmittelbar benachbart zum Schneidelement 20 ist dabei eine Öffnung 12 vorgesehen, welche eine Verbindung zwischen dem Hohlraum 25 und der Außenseite bereitstellt (vgl. Fig. 6). Somit kann durch Anlegen eines Unterdrucks an den Hohlraum 5 Gewebe, Blut und dgl. während der Operation aus dem Operationsbereich durch die Öffnung 2 und den Hohlraum 25 abgesaugt werden.

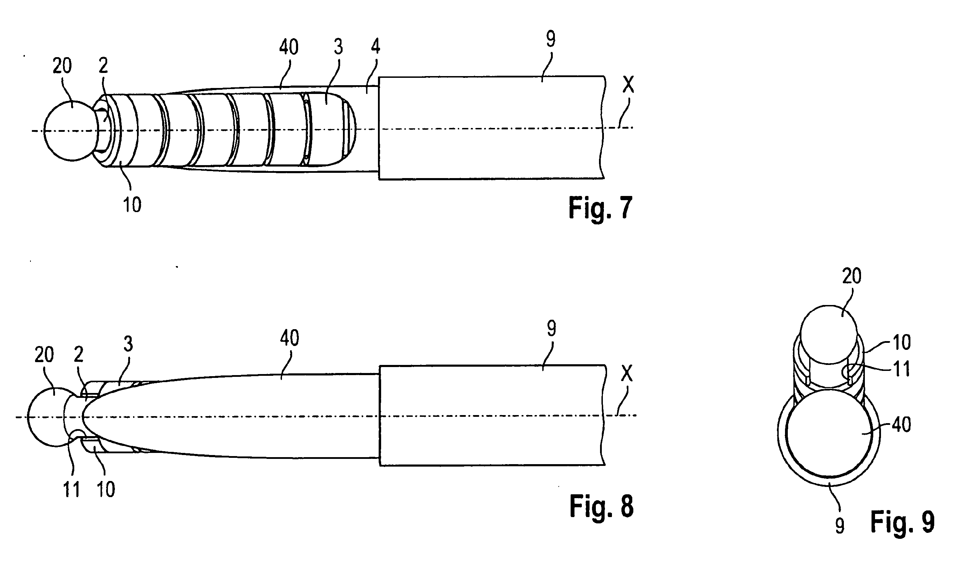

Weiterhin ist eine Lagerhülse 10 vorgesehen, welche am ersten Endbereich 31 des Zwischenrohrs 3 angeordnet ist. Wie insbesondere aus Fig. 6 ersichtlich ist, ist die Lagerhülse 10 am distalen Ende des Zwischenrohrs 3 angeordnet. Dies kann beispielsweise mittels einer Schweißverbindung erfolgen. Die Lagerhülse 10 weist einen durchgehenden Längsschlitz 11 auf, welcher die Verbindung zwischen der Öffnung 12 und der Umgebung am distalen Ende des chirurgischen Instruments herstellt (vgl. Fig. 8 und 9).

Weiterhin ist die Führungszunge 40 derart gebogen, dass die Führungszunge vollständig in einem durch einen Außenumfang des Führungsrohres 4 definierten, zylindrischen Umrisskörper 4' angeordnet ist. Der Umrisskörper 4' ist in Fig. 6 durch gestrichelte Linien angedeutet. Dadurch wird eine sichere Einführbarkeit des erfindungsgemäßen chirurgischen Instruments sichergestellt.

Die Funktion des erfindungsgemäßen chirurgischen Instruments ist dabei wie folgt. Das chirurgische Instrument 1 wird über die Kupplung 5 mit einem nicht gezeigten Antrieb verbunden. Der Antrieb kann beispielsweise in einem Handgriff oder dgl. vorgesehen sein. Dabei weist das chirurgische Instrument 1 die in den Fig. 1 und 3 gezeigte zurückgezogene Position auf. D.h., zwischen dem Schneidenhalter 6 und dem Rohrhalter 7 ist in Längsrichtung X ein Abstand C vorhanden (siehe Fig. 3). Wie insbesondere aus Fig. 3 ersichtlich ist, ist in dieser zurückgezogenen Position die Führungszunge 40 des Führungsrohrs 4 völlig frei. Da, wie insbesondere aus Fig. 8 ersichtlich ist, die Führungszunge 40 eine abgerundete Spitze aufweist, kann diese leicht an eine Operationsstelle, beispielsweise in einem Knie, eingeführt werden. Vorzugsweise wurde hierbei schon in einem ersten Schritt das Zugangsrohr 9 positioniert, so dass das erfindungsgemäße chirurgische Instrument 1 lediglich in das Zugangsrohr 9 eingeführt werden muss und dann weiter zur Operationsstelle geschoben werden kann. Dies wird beispielsweise unter Röntgen- oder Ultraschall-Kontrolle oder durch eine in die Operationsstelle schon eingeführte Kamera überwacht.

Wenn die richtige Position gefunden ist, wird das Schneidrohr 2 mitsamt dem Zwischenrohr 3 aus dem Führungsrohr 4 vorgeschoben. Dies kann dadurch erfolgen, dass der Rohrhalter 7 mit einer Hand festgehalten wird und der Schneidenhalter 6 in Richtung des Pfeils A entlang der Stifte 8 vorgeschoben wird (vgl. Fig. 3). Eine vollständig vorgeschobene Position ist dabei in den Fig. 2, 4 und 6 gezeigt. Dadurch steht das Schneidenelement 20 frei von der Führungszunge 40 vor, so dass Körpermaterial abgetragen werden kann, sobald das Schneidrohr 2 angetrieben wird. Die Schlitze 24 im Schneidrohr 2 und die Schlitze 34 im Zwischenrohr 3 sind dabei derart gebildet, dass das Schneidrohr bzw. das Zwischenrohr eine Flexibilität derart aufweisen, dass diese in Längsrichtung X entlang der Führungszunge 40 verschoben werden können, ohne dass es hierbei zu Beschädigungen im Bereich der Schlitze kommt. Das dabei abgetragene Körpermaterial wird dann über den Schlitz 11 in der Lagerhülse 10 und die Öffnung 12 im Schneidrohr 2 in den Hohlraum 25 des Schneidrohrs eingesaugt und durch die Kupplung 5 abgeführt. Zur Sicherheit wird dabei eine Relativposition zwischen dem Schneidenhalter 6 und dem Rohrhalter 7 mittels der Verriegelungseinrichtung 14 fixiert. Dabei übernimmt die Führungszunge 40 die exakte Positionierung des Schneidenelements 20 am ersten Endbereich 21 des Schneidrohrs 2. Da die Führungszunge 40 nur an im Wesentlichen einem Seitenumfang des Führungsrohrs 4 gebildet ist, liegt ein Großteil des Zwischenrohrs 3 und das Schneidenelement 2 am ersten Endbereich 41 des Führungsrohrs 4 frei (vgl. Fig. 7). Hierdurch wird während des Vorschiebevorgangs und während des Bearbeitungsvorgangs eine Behinderung des rotierenden Schneidrohrs 2 bzw. des nicht rotierenden Zwischenrohrs 3 vermieden.

Somit wird erfindungsgemäß ein rotierendes chirurgisches Instrument 1 bereitgestellt, welches mittels eines distalen flexiblen Endbereichs auch eine Bearbeitung an schwierig zu erreichenden Körperstellen ermöglicht. Das erfindungsgemäße Instrument eignet sich besonders zur Erzeugung von Hohlräumen.

Nach einer erfolgten Operation kann das erfindungsgemäße chirurgische Instrument 1 auch leicht zerlegt werden, indem die Kupplung 5 mitsamt dem Schneidrohr 2 aus dem Zwischenrohr 3 herausgezogen wird und der Schneidehalter 6 mitsamt dem Zwischenrohr 3 aus dem Führungsrohr 4 herausgezogen wird. Dadurch können die Einzelteile, welche vorzugsweise aus Edelstahl hergestellt sind, leicht gereinigt werden und anschließend wieder zusammengesetzt werden.

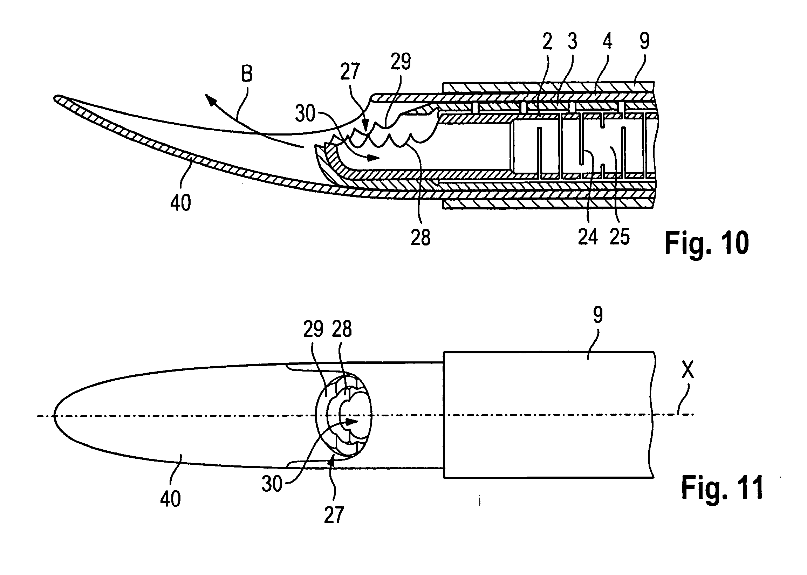

Ein zweites Ausführungsbeispiel ist in den Fig. 10 und 11 gezeigt, wobei gleiche bzw. funktional gleiche Teile mit den gleichen Bezugszeichen wie im vorhergehenden Ausführungsbeispiel bezeichnet sind. Beim ersten Ausführungsbeispiel war das Schneidelement 20 ein Fräser, mit welchem insbesondere härtere Körpermaterialien, wie z.B. Knochen, bearbeitet werden können. Beim zweiten Ausführungsbeispiel ist das Schneidelement 20 ein U-förmiger Schneidkopf 27 mit einem stillstehenden Teil 29 und einem rotierenden Teil 28 sowie mit einer Vielzahl von Schneidzähnen. Alternativ kann der Schneidkopf auch eine einzige U-förmig umlaufende Schneide aufweisen. Der Schneidkopf 28 ist insbesondere zum Abtragen von weicherem Gewebe ausgelegt. Am Schneidkopf 28 ist eine Absaugöffnung 29 vorgesehen, welche eine Verbindung zwischen dem Hohlraum 25 im Schneidrohr 2 und der Umgebung herstellt. Somit kann abgetrenntes Gewebe durch die Absaugöffnung 29 in das Innere des Schneidrohrs 2 eingesaugt werden. In Fig. 10 ist durch den Pfeil B nochmals angedeutet, wie der Schneidkopf 28 gemeinsam mit dem Zwischenrohr 3 aus dem Führungsrohr 4 herausgeschoben werden kann, wobei die Führungszunge 40 die Richtung, in welche der Schneidkopf herausgeschoben wird, vorgibt.

### Bezugszeichenliste

- 1: chirurgisches Instrument
- 2: Schneidrohr
- 3: Zwischenrohr
- 4: Führungsrohr
- 4': Umrisskörper
- 5: Kupplung
- 6: Schneidenhalter
- 7: Rohrhalter
- 8: Stift
- 9: Zugangsrohr
- 10: Lagerhülse
- 11: Schlitz
- 12: Absaugöffnung
- 14: Verriegelungseinrichtung
- 20: Schneidelement
- 21: erster Endbereich
- 22: zweiter Endbereich
- 23: erster flexibler Teilbereich
- 24: Schlitz
- 25: Hohlraum
- 26: Schweißverbindung
- 27: Schneidkopf
- 28: rotierender Teil
- 29: stillstehender Teil
- 30: Absaugöffnung
- 31: erster Endbereich
- 32: zweiter Endbereich
- 33: zweiter flexibler Teilbereich
- 34: Schlitz
- 35: Schweißverbindung
- 40: Führungszunge
- 41: erster Endbereich
- 42: zweiter Endbereich
- 44: Schweißverbindung
- A: Schieberichtung
- B: Ausschieberichtung
- C: Abstand
- X: Längsachse

## Patentansprüche

1. Chirurgisches Instrument, umfassend:
- ein rotierbares Schneidrohr (2) mit einem Schneidelement (20; 28) an einem ersten Endbereich (21), und
- ein das Schneidrohr (2) umgebendes Zwischenrohr (3),
- ein das Zwischenrohr (3) umgebendes Führungsrohr (4),
- einen Rohrhalter (7), an welchem das Führungsrohr (4) fixiert ist, und
- einen Schneidenhalter (6), an welchem das Zwischenrohr (3) fixiert ist,
- wobei das Schneidrohr (2) und das Zwischenrohr (3) relativ zum Führungsrohr (4) in Längsrichtung (X) des chirurgischen Instruments verschiebbar sind, und
- wobei das Schneidrohr (2) eingerichtet ist, an einem zweiten Endbereich (22) mit einem Antrieb verbunden zu werden, **dadurch gekennzeichnet, dass**
- das Schneidrohr (2) einen ersten flexiblen Teilbereich (23) aufweist und das Zwischenrohr (3) einen zweiten flexiblen Teilbereich (33) aufweist, welcher an einer Position entsprechend einer Position des ersten flexiblen Teilbereichs (23) des Schneidrohrs (2) vorgesehen ist;
- wobei das Führungsrohr (4) an einem ersten Endbereich (41) eine bogenförmige Führungszunge (40) aufweist, welche den ersten und zweiten flexiblen Teilbereich des Schneidrohrs (2) und des Zwischenrohrs 3 während eines Verschiebevorgangs führt und auslenkt

2. Instrument nach Anspruch 1, **dadurch gekennzeichnet, dass** das Schneidrohr (2) eine Absaugöffnung (12) umfasst, welche insbesondere am oder benachbart zum Schneidelement (20; 28) angeordnet ist.

3. Instrument nach einem der vorhergehenden Ansprüche, **dadurch gekennzeichnet, dass** die Führungszunge (40) vollständig in einem durch einen Außenumfang des Führungsrohrs (4) definierten zylindrischen Umrisskörper (4') angeordnet ist.

4. Instrument nach einem der vorhergehenden Ansprüche, **dadurch gekennzeichnet, dass** der Schneidenhalter (6) mit dem Rohrhalter (7) verdrehsicher verbunden ist.

5. Instrument nach einem der vorhergehenden Ansprüche, **dadurch gekennzeichnet, dass** zwischen dem Schneidenhalter (6) und dem Rohrhalter (7) eine lösbare Verriegelungseinrichtung (14) vorgesehen ist.

6. Instrument nach einem der vorhergehenden Ansprüche, **dadurch gekennzeichnet, dass** der erste flexible Teilbereich (23) des Schneidrohrs (2) eine Vielzahl von Schlitzen (24) aufweist und/oder dass der zweite flexible Teilbereich (33) des Zwischenrohrs (3) eine Vielzahl von Schlitzen (34) aufweist.

7. Instrument nach einem der vorhergehenden Ansprüche, **dadurch gekennzeichnet, dass** die Führungszunge (40) eine abgerundete Spitze aufweist.

8. Instrument nach einem der vorhergehenden Ansprüche, **dadurch gekennzeichnet, dass** sich der erste flexible Teilbereich (23) direkt an das Schneidelement (20; 28) des Schneidrohrs (2) anschließt.

9. Instrument nach einem der vorhergehenden Ansprüche, ferner umfassend eine Lagerhülse (10), welche am ersten Endbereich (31) des Zwischenrohrs (3) angeordnet ist, um das Schneidrohr (2) rotierbar zu lagern.

10. Instrument nach einem der vorhergehenden Ansprüche, **dadurch gekennzeichnet, dass** das Schneidrohr (2) am zweiten Endbereich (22) mit einer Kupplung (5) verbunden ist, wobei die Kupplung (5) eingerichtet ist, ein Drehmoment zum Antreiben des Schneidrohrs (2) zu empfangen.

11. Instrument nach einem der vorhergehenden Ansprüche, **dadurch gekennzeichnet, dass** das Schneidrohr (2) vom Zwischenrohr (3) und das Zwischenrohr (3) vom Führungsrohr (4) zerlegbar ist.

12. Instrument nach einem der vorhergehenden Ansprüche, **dadurch gekennzeichnet, dass** das Schneidelement (20) ein Fräser ist oder dass das Schneidelement (20) ein Schneidkopf (28) zum Schneiden von Gewebe oder dgl. ist.

13. Instrument nach einem der vorhergehenden Ansprüche, **dadurch gekennzeichnet, dass** das Schneidrohr (2) und/oder das Zwischenrohr (3) und/oder das Führungsrohr (4) aus Edelstahl hergestellt sind.

14. Instrument nach einem der vorhergehenden Ansprüche, ferner umfassend ein Zugangsrohr (9), welches das Führungsrohr (4) umgibt.

15. Instrument nach einem der vorhergehenden Ansprüche, **dadurch gekennzeichnet, dass** die Führungszunge (40) und das Führungsrohr (4) als einstückiges Bauteil vorgesehen sind.

## Claims

1. Surgical instrument comprising:
- a rotatable cutting tube (2) with a cutting element (20; 28) at a first end area (21), and
- an intermediate tube (3) surrounding the cutting tube (2),
- a guide tube (4) surrounding the intermediate tube (3),
- a tube holder (7) at which the guide tube (4) is fixed, and
- a blade holder (6) at which the intermediate tube (3) is fixed,
- the cutting tube (2) and the intermediate tube (3) being displaceable relative to the guide tube (4) in a longitudinal direction (X) of the surgical instrument, and
- the cutting tube (2) being configured to be connected at a second end area (22) to a drive,
**characterized in that**
- the cutting tube (2) comprises a first flexible sub-area (23) and the intermediate tube (3) comprises a second flexible sub-area (33) which is provided at a position corresponding to a position of the first flexible sub-area (23) of the cutting tube (2),
- the guide tube (4) at a first end area (41) comprising an arcuate guide tongue (41) which guides and deflects the first and second flexible sub-area of the cutting tube (2) and of the intermediate tube (3) during a displacement process.

2. Instrument according to claim 1, **characterized in that** the cutting tube (2) comprises a suction port (12) which is arranged particularly at or adjacent to the cutting element (20; 28).

3. Instrument according to any one of the preceding claims, **characterized in that** the guide tongue (40) is arranged completely within a cylindrical contour body (4') defined by an outer circumference of the guide tube (4).

4. Instrument according to any one of the preceding claims, **characterized in that** the blade holder (6) is non-rotationally connected to the tube holder (7).

5. Instrument according to any one of the preceding claims, **characterized in that** a detachable locking means (14) is provided between the blade holder (6) and the tube holder (7).

6. Instrument according to any one of the preceding claims, **characterized in that** the first flexible sub-area (23) of the cutting tube (2) comprises a plurality of slits (24) and/or that the second flexible sub-area (33) of the intermediate tube (3) comprises a plurality of slits (34).

7. Instrument according to any one of the preceding claims, **characterized in that** the guide tongue (40) has a rounded tip.

8. Instrument according to any one of the preceding claims, **characterized in that** the first flexible sub-area (23) directly follows the cutting element (20; 28) of the cutting tube (2).

9. Instrument according to any one of the preceding claims, further comprising a bearing sleeve (10) which is arranged at the first end area (31) of the intermediate tube (3) to rotatably support the cutting tube (2).

10. Instrument according to any one of the preceding claims, **characterized in that** the cutting tube (2) is connected at the second end area (22) to a coupling (5), the coupling (5) being configured to receive a torque for driving the cutting tube (2).

11. Instrument according to any one of the preceding claims, **characterized in that** the cutting tube (2) is dismountable from the intermediate tube (3) and the intermediate tube (3) from the guide tube (4).

12. Instrument according to any one of the preceding claims, **characterized in that** the cutting element (20) is a milling cutter, or that the cutting element (20) is a cutting head (28) for cutting tissue, or the like.

13. Instrument according to any one of the preceding claims, **characterized in that** the cutting tube (2) and/or the intermediate tube (3) and/or the guide tube (4) are made from stainless steel.

14. Instrument according to any one of the preceding claims, further comprising an access tube (9) which surrounds the guide tube (4).

15. Instrument according to any one of the preceding claims, **characterized in that** the guide tongue (40) and the guide tube (4) are provided as an integral component.

## Revendications

1. Instrument chirurgical, comprenant :
- un tube de coupe rotatif (2) pourvu d'un élément de coupe (20 ; 28) à une première zone d'extrémité (21), et
- un tube intermédiaire (3) entourant le tube de coupe (2),
- un tube de guidage (4) entourant le tube intermédiaire (3),
- un support de tube (7) auquel le tube de guidage (4) est fixé, et
- un support de lame (6) auquel le tube intermédiaire (3) est fixé,
- le tube de coupe (2) et le tube intermédiaire (3) étant déplaçables par rapport au tube de guidage (4) dans la direction longitudinale (X) de l'instrument chirurgical, et
- le tube de coupe (2) étant conçu pour être relié à un entraînement à une deuxième zone d'extrémité (22),
**caractérisé en ce que**
- le tube de coupe (2) présente une première partie flexible (23) et le tube intermédiaire (3) présente une deuxième partie flexible (33), laquelle est prévue à une position qui correspond à une position de la première partie flexible (23) du tube de coupe (2) ;
- le tube de guidage (4) présentant à une première zone d'extrémité (41) une languette de guidage arquée (40) qui guide et dévie la première et la deuxième partie flexible du tube de coupe (2) et du tube intermédiaire 3 pendant un déplacement.

2. Instrument selon la revendication 1, **caractérisé en ce que** le tube de coupe (2) comprend une ouverture d'aspiration (12), laquelle est en particulier disposée sur ou au voisinage de l'élément de coupe (20 ; 28).

3. Instrument selon l'une des revendications précédentes, **caractérisé en ce que** la languette de guidage (40) est entièrement disposée dans un corps de contour cylindrique (4') défini par un pourtour extérieur du tube de guidage (4).

4. Instrument selon l'une des revendications précédentes, **caractérisé en ce que** le support de lame (6) est relié sans possibilité de rotation au support de tube (7).

5. Instrument selon l'une des revendications précédentes, **caractérisé en ce qu'**un dispositif de verrouillage (14) libérable est prévu entre le support de lame (6) et le support de tube (7).

6. Instrument selon l'une des revendications précédentes, **caractérisé en ce que** la première partie flexible (23) du tube de coupe (2) présente une pluralité de fentes (24) et/ou que la deuxième partie flexible (33) du tube intermédiaire (3) présente une pluralité de fentes (34).

7. Instrument selon l'une des revendications précédentes, **caractérisé en ce que** la languette de guidage (40) présente une pointe arrondie.

8. Instrument selon l'une des revendications précédentes, **caractérisé en ce que** la première partie flexible (23) est directement adjacente à l'élément de coupe (20 ; 28) du tube de coupe (2).

9. Instrument selon l'une des revendications précédentes, comprenant en outre une douille de palier (10), laquelle est disposée à la première zone d'extrémité (31) du tube intermédiaire (3) pour loger le tube de coupe (2) à rotation.

10. Instrument selon l'une des revendications précédentes, **caractérisé en ce que** le tube de coupe (2) est relié à la deuxième zone d'extrémité (22) à un accouplement (5), l'accouplement (5) étant conçu pour recevoir un couple pour entraîner le tube de coupe (2).

11. Instrument selon l'une des revendications précédentes, **caractérisé en ce que** le tube de coupe (2) est démontable du tube intermédiaire (3) et le tube intermédiaire (3) du tube de guidage (4).

12. Instrument selon l'une des revendications précédentes, **caractérisé en ce que** l'élément de coupe (20) est une fraise ou que l'élément de coupe (20) est une tête de coupe (28) pour couper un tissu ou analogue.

13. Instrument selon l'une des revendications précédentes, **caractérisé en ce que** le tube de coupe (2) et/ou le tube intermédiaire (3) et/ou le tube de guidage (4) sont fabriqués en acier inoxydable.

14. Instrument selon l'une des revendications précédentes, comprenant en outre un tube d'accès (9) qui entoure le tube de guidage (4).

15. Instrument selon l'une des revendications précédentes, **caractérisé en ce que** la languette de guidage (40) et le tube de guidage (4) sont prévus sous la forme d'un composant d'une seule pièce.
